(19) **Europäisches Patentamt / European Patent Office / Office européen des brevets**

(11) **EP 4 475 760 B1**

(12) **FASCICULE DE BREVET EUROPEEN**

(45) Date de publication et mention de la délivrance du brevet:
**18.03.2026 Bulletin 2026/12**

(21) Numéro de dépôt: **23703215.6**

(22) Date de dépôt: **06.02.2023**

(51) Classification Internationale des Brevets (IPC):
***A61B 5/12*** *(2006.01)*

(52) Classification Coopérative des Brevets (CPC):
**A61B 5/123**

(86) Numéro de dépôt international:
**PCT/EP2023/052758**

(87) Numéro de publication internationale:
**WO 2023/152065 (17.08.2023 Gazette 2023/33)**

(54) **PROCÉDÉ DE TEST AUDIOMÉTRIQUE ET DISPOSITIF ÉLECTRONIQUE ASSOCIÉ**

VERFAHREN ZUR AUDIOMETRISCHEN PRÜFUNG UND ZUGEHÖRIGE ELEKTRONISCHE VORRICHTUNG

METHOD FOR AUDIOMETRIC TESTING AND ASSOCIATED ELECTRONIC DEVICE

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats de validation désignés:
**MA TN**

(30) Priorité: **08.02.2022 FR 2201113**

(43) Date de publication de la demande:
**18.12.2024 Bulletin 2024/51**

(73) Titulaire: **My Medical Assistant
51100 Reims (FR)**

(72) Inventeur: **WALLAERT, Nicolas
51100 Reims (FR)**

(74) Mandataire: **Radzimski, Eric
Cabinet Aubenard
21, boulevard Carnot
21000 Dijon (FR)**

(56) Documents cités:
**US-A1- 2010 257 128**

- **TREVOR J LARSEN ET AL: "Accelerating Psychometric Screening Tests With Bayesian Active Differential Selection", ARXIV.ORG, CORNELL UNIVERSITY LIBRARY, 201 OLIN LIBRARY CORNELL UNIVERSITY ITHACA, NY 14853, 4 February 2020 (2020-02-04), XP081592763**
- **COX MARCO ET AL: "Bayesian Pure-Tone Audiometry Through Active Learning Under Informed Priors", FRONTIERS IN DIGITAL HEALTH, 13 August 2021 (2021-08-13), pages 723348 - 723348, XP055945404, Retrieved from the Internet <URL:https://www.ncbi.nlm.nih.gov/pmc/articles/PMC8521968/pdf/fdgth-03-723348.pdf> [retrieved on 20220722], DOI: 10.3389/fdgth.2021.723348**

## Description

L'invention concerne l'audiométrie tonale. Des méthodes de test audiométriques sont connues de US-2010/257128-A1, TREVOR J LARSEN ET AL: "Accelerating Psychometric Screening Tests With Bayesian Active Differential Selection", CORNELL UNIVERSITY LIBRARY, février 2020, et Cox Marco ET AL: "Bayesian Pure-Tone Audiometry Through Active Learning Under Informed Priors", Frontiers in digital health, août 2021, DOI: 10.3389/fdgth.2021.723348.

L'audiométrie tonale permet de mesurer de façon discrète, par voie osseuse et par voie aérienne, un seuil d'audition pour une plage sonore s'étendant, par exemple, de 125 à 8000 Hertz (de manière habituelle, les Hertz sont notés Hz dans la suite) pour les fréquences conversationnelles en transmission aérienne et de 250 à 6000Hz en transmission osseuse. Lors d'une audiométrie haute fréquence, la plage sonore testée peut être étendue jusqu'à 20000 Hertz.

Une série de son est appliquée à l'oreille testée par exemple par l'intermédiaire d'un casque en conduction aérienne ou d'un vibrateur en conduction osseuse. Il est demandé au patient d'appuyer sur le bouton réponse dès lors qu'il entend un son.

Il a été proposé d'employer un processus gaussien pour déterminer les sons à présenter au patient de manière à obtenir, rapidement et précisément, le seuil d'audition pour l'ensemble de la plage sonore.

Par exemple, une telle approche a été publiée dans l'article de Song XD, Wallace BM, Gardner JR, Ledbetter NM, Weinberger KQ, Barbour DL. Fast, « Continuous Audiogram Estimation using Machine Learning ». Ear Hear. 2015;36(6):e326-35.

Il existe un besoin de rendre une telle approche applicable à un large public et pour l'intégralité des données recueillies lors d'un examen audiométrique tonale (courbe aérienne et osseuse).

Dans ce but, l'invention concerne un procédé de test audiométrique d'une oreille (d'un patient, en transmission aérienne ou osseuse) (dans une plage sonore) (bien entendu, le même test audiométrique peut tester en outre l'oreille controlatérale simultanément. L'audition des deux oreilles combinées du patient est alors testée. Ainsi le test audiométrique, selon l'invention peut être réalisé en champs libre) caractérisé en ce qu'il comporte les étapes suivantes :

- Détermination d'une séquence comprenant :

  - Des premiers sons (dans la plage sonore) appliqués à l'oreille,
  - Pour chacun des premiers sons, une première information selon laquelle les premiers sons sont entendus ou pas (par le patient),

- Répétition des étapes suivantes un nombre de fois jusqu'à ce qu'une condition soit remplie :

  - Détermination d'un deuxième son ayant une deuxième fréquence et une deuxième intensité à partir d'un processus gaussien prenant la séquence comme données observées,
  - Détermination, s'il existe, d'un troisième son de (autrement dit : dans) la séquence présentant une troisième intensité inférieure à la deuxième intensité et supérieure à un deuxième seuil et (le troisième son) présentant une troisième fréquence, la différence entre la troisième fréquence et la deuxième fréquence étant inférieure à un seuil de fréquence (le seuil de fréquence peut être fixe ou variable, par exemple fixé à ½ octave), et la troisième intensité étant maximum parmi tous les sons de la séquence dont la fréquence a une différence avec la deuxième fréquence inférieure au seuil de fréquence,
  - Application à l'oreille d'un quatrième son ayant une quatrième fréquence et une quatrième intensité, où :

    - Si la deuxième intensité est supérieure au deuxième seuil, et s'il existe un troisième son, la quatrième fréquence est égale à la deuxième fréquence et la quatrième intensité est égale à la troisième intensité augmentée d'un incrément prédéfini,
    - Sinon (c'est-à-dire, la deuxième intensité est inférieure au deuxième seuil ou il n'existe pas de troisième son) alors le quatrième son est identique au deuxième son,

  - Acquisition d'une quatrième information selon laquelle le quatrième son est entendu ou pas (par le patient)
  - Mise à jour de la séquence à partir de la quatrième information et du quatrième son (autrement dit : la quatrième information et le quatrième son sont ajoutées à la séquence).

On évite ainsi d'augmenter trop vite l'intensité au-delà du deuxième seuil autour de chaque fréquence, pour prévenir les sur-stimulations et prendre en compte les phénomènes de recrutement (augmentation anormalement rapide de la sonie pouvant subvenir pour les surdités neurosensorielles). Ainsi, le procédé de test audiométrique selon l'invention est adapté, même en cas de surdité importante.

Selon un mode réalisation, de manière connue, le processus gaussien, défini à partir des données observées, des probabilités d'entendre chaque son dans la plage sonore et une incertitude sur ces probabilités et le deuxième son a une deuxième fréquence et une deuxième intensité maximisant une réduction de cette incertitude.

Selon un mode de réalisation, le processus gaussien est mis en œuvre comme décrit dans l'article de Schlittenlacher J, Turner RE, Moore BCJ. « Audiogram estimation using Bayesian active learning » (J Acoust Soc Am. 2018;144(1):421). Notamment le processus a les caractéristiques suivantes.

Sur le plan fréquentiel, on utilise par exemple un noyau exponentiel qui permet de rendre compte du fait que les seuils, à des fréquences adjacentes, sont corrélés, par exemple :

$$k_{\mathrm{SE}}(x, x') = \sigma^2 \exp\left(-\frac{(x-x')^2}{2\ell^2}\right)$$

où le premier terme à gauche ($k_{\mathrm{SE}}(x,x')$) correspond au noyau exponentiel carré. Le noyau est utilisé pour calculer une matrice de covariance permettant ensuite de créer des fonctions. Le terme $\sigma$ correspond à la variance, c'est-à-dire la distance des fonctions à la moyenne ou la profondeur de modulation, 1 désigne la longueur des ondulations, x et x' désigne toutes les paires de points possibles.

Sur le plan de l'intensité, on utilise par exemple un noyau linéaire qui permet de rendre compte du fait que la probabilité qu'un son soit entendu augmente lorsque l'on augmente l'intensité, par exemple :

$$k(x_i, x_j) = \sigma_0^2 + x_i \cdot x_j$$

où le premier terme à gauche ($k(x_i,x_j)$) correspond au noyau linéaire, ($\sigma_0$ correspond à la variance de l'intercept de la droite, c'est-à-dire qu'une grande valeur correspond à une grande variabilité sur les intercepts, $x_i$ et $x_j$ désigne toutes les paires de points possibles.

Le deuxième son est par exemple choisi de manière à maximiser la fonction suivante qui mesure l'information mutuelle entre la réponse attendue et l'estimation du processus gaussien:

$$I(y^*; \theta|x^*) = H(y^*|x^*, D) - E_{\theta \sim p(\theta|D)}(H[y^*|x^*, \theta]),$$

où le premier terme à droite correspond à l'entropie de la réponse attendue et le second terme est l'entropie conditionnelle attendue de la réponse compte tenu de l'estimation de la fonction du processus gaussien, H désigne l'entropie de Shannon, D les réponses déjà obtenues (C'est-à-dire : les informations dans la séquence), x* la fréquence et l'intensité du signal du deuxième son, y* la réponse attendue, $\theta$ la fonction du processus gaussien.

Par exemple, la condition est remplie si :

- L'amplitude de l'intervalle d'incertitude estimé par le processus gaussien (L'intervalle d'incertitude est déterminé comme étant la zone couverte par un écart-type autour de l'audiogramme estimé par le processus gaussien, c'est-à-dire où la probabilité est de 50%) est inférieure à une valeur seuil (par exemple 6 dB HL, ou plus généralement une valeur seuil comprise entre 1 et 10 dB), ou
- Le nombre de fois dépasse un seuil de répétition (par exemple compris entre 15 et 100 fois).

Selon un mode de réalisation, l'étape de détermination d'une séquence comprend la répétition des étapes suivantes jusqu'à ce qu'une cinquième fréquence ait pris toutes les valeurs de fréquence d'une série de fréquences :

- Détermination d'un cinquième son présentant (autrement dit : ayant) la cinquième fréquence, et une cinquième intensité,
- Application à l'oreille du cinquième son,
- Acquisition d'une cinquième information selon laquelle le cinquième son est entendu ou pas (par le patient),
- Mise à jour de la séquence à partir du cinquième son et de la cinquième information.

En variante, la séquence peut être réceptionnée ou lue en mémoire.

Selon un mode de réalisation, l'étape de détermination d'une séquence comprend la première répétition des étapes suivantes jusqu'à ce qu'une cinquième fréquence ait pris toutes les valeurs de fréquence d'une série de fréquences :

- Détermination d'un cinquième son présentant (autrement dit : ayant) la cinquième fréquence, et une cinquième intensité,
- Une (deuxième) répétition des (cinq) étapes suivantes jusqu'à ce que le cinquième son ait été entendu une fois et pas entendu une fois ou jusqu'à ce que la cinquième intensité atteigne une intensité maximale, par exemple 90 dB HL (les décibels perte auditive, en anglais « Hearing Loss», sont notés dB HL) ou une intensité minimale, par exemple -20 dB HL :

  - Application à l'oreille (210) du cinquième son,
  - Acquisition d'une cinquième information selon laquelle le cinquième son est entendu ou pas (par le patient),
  - Mise à jour de la séquence à partir du cinquième son et de la cinquième information
  - Si le cinquième son est entendu, décrémentation de la cinquième intensité d'une valeur de décrémentation, par exemple de 20 dB HL.
  - Si le cinquième son n'est pas entendu, incrémentation de la cinquième intensité d'une valeur d'incrémentation, par exemple de 10 dB HL.

- Affectation de la cinquième intensité par (c'est-à-dire que la cinquième intensité prend la valeur suivante):

  - Si un cinquième son a été entendu (par le patient) (durant la deuxième répétition des étapes), une intensité minimum où le cinquième son a été entendu (à la cinquième fréquence) auquel on ajoute une marge d'intensité, par exemple de 10 dB HL.
  - Si le cinquième son n'a pas été entendu (par le patient) (durant la deuxième répétition des étapes), l'intensité maximale, par exemple de 90 dB HL.
  - Augmentation ou diminution de la cinquième fréquence d'un incrément de fréquence, (de manière à ce que la cinquième fréquence prenne toutes les valeurs de fréquence de la série de fréquences), par exemple une augmentation ou une diminution de 500 Hz.

En variante, le procédé de test audiométrique est réalisé en conduction osseuse (i.e. : le quatrième son est appliqué à l'oreille en conduction osseuse) et comprend, préalablement à l'étape de détermination d'une séquence :

- Une détermination d'un audiogramme en conduction aérienne comprenant une intensité aérienne pour chaque fréquence d'une série de fréquence (l'intensité aérienne pour chaque fréquence est l'intensité minimum où le son est entendu à cette fréquence),
- Une initialisation d'un état à une première valeur, (l'état est par exemple mémorisé en mémoire),
et dans lequel l'étape de détermination d'une séquence comprend la première répétition des étapes suivantes jusqu'à ce qu'une cinquième fréquence ait pris toutes les valeurs de fréquence de la série de fréquences, la cinquième fréquence étant initialisée à la première fréquence de la série de fréquence :

  - Si l'état a la première valeur :

    - Détermination d'un cinquième son présentant la cinquième fréquence, et une cinquième intensité égale à l'intensité aérienne pour la cinquième fréquence dans l'audiogramme en conduction aérienne auquel on ajoute une intensité additionnelle, par exemple de 5dB,
    - Application à l'oreille (210) du cinquième son par conduction osseuse (un masquage du cinquième son peut être appliqué à l'oreille controlatérale),
    - Acquisition d'une cinquième l'information selon laquelle le cinquième son est entendu ou pas,
    - Mise à jour de la séquence à partir du cinquième son et de la cinquième information,
    - Si le cinquième son est entendu, affectation de l'état par une deuxième valeur.

  - Si l'état à la deuxième valeur :

    - Une répétition des (cinq) étapes suivantes jusqu'à ce que le cinquième son ait été entendu une fois et pas entendu une fois ou jusqu'à ce que la cinquième intensité atteigne une intensité maximale, ou une intensité minimale :
    - Application à l'oreille (210) du cinquième son,
    - Acquisition d'une cinquième information selon laquelle le cinquième son est entendu ou pas,
    - Mise à jour de la séquence à partir du cinquième son et de la cinquième information,
    - Si le cinquième son est entendu, décrémentation de la cinquième intensité d'une valeur de décrémentation.
    - Si le cinquième son n'est pas entendu, incrémentation de la cinquième intensité d'une valeur d'incrémentation.

- Affectation de la cinquième intensité par :

  - Si le cinquième son a été entendu, une intensité minimum où le cinquième son a été entendu auquel on ajoute une marge d'intensité,
  - Si le cinquième son n'a pas été entendu, une intensité maximale,

- Augmentation de la cinquième fréquence d'un incrément de fréquence (de manière à ce que la cinquième fréquence prenne toutes les valeurs de fréquence de la série de fréquences).

Lorsque la cinquième fréquence est une fréquence d'extrémité (par exemple les fréquences de 8000 Hz et 125 Hz sont des fréquences d'extrémité lorsque la série de fréquence est constituée des fréquences suivantes : 1000, 1500, 2000, 3000, 4000, 6000, 8000, 750, 500, 250 et 125 Hz) de la série de fréquences, la valeur d'incrémentation (et/ou la valeur de décrémentation, respectivement) peut prendre une valeur inférieure, par exemple 10 dB HL, à la valeur de décrémentation (et/ou la valeur de décrémentation, respectivement) lorsque la fréquence est une autre fréquence (qu'une fréquence d'extrémité).

Lorsque la cinquième intensité dépasse un cinquième seuil, par exemple 80 dB, la valeur d'incrémentation prend une valeur inférieure, par exemple 5 dB HL, à la valeur d'incrémentation (utilisée) lorsque la cinquième intensité est en dessous du cinquième seuil.

On évite ainsi les phénomènes de sur-stimulation chez des patients présentant un recrutement de sonie pouvant survenir durant l'étape de détermination de la séquence.

La série de fréquence peut être constituée des fréquences suivantes :1000, 2000, 4000, 8000, 500, et 250.Hz Toutefois, une série avec un nombre plus important de fréquences présente l'avantage de faciliter la correction des erreurs de réponse du sujet (e.g. lorsqu'un patient n'indique pas qu'il a entendu un son alors qu'il l'a entendu). Préférentiellement, donc, (pour une audiométrie par voie aérienne, par exemple) la série de fréquence est constituée des fréquences suivantes : 1000, 1500, 2000, 3000, 4000, 6000, 8000, 750, 500, 250 et 125 Hz en conduction aérienne et 1000, 1500, 2000, 3000, 4000, 6000, 750, 500 et 250 Hz en conduction osseuse.

Selon un mode de réalisation, durant l'étape de détermination du deuxième son, le deuxième son est déterminé, à partir du processus gaussien, dans une plage de fréquences sonores (autrement dit : le processus gaussien défini des probabilités d'entendre chaque son dans la plage de fréquences sonores et une incertitude sur ces probabilités, et le deuxième son maximise une réduction de cette incertitude), et la plage de fréquences sonores exclue une plage de fréquences d'exclusions où aucun son (i .e. : aucun son émis, ayant une fréquence dans la plage de fréquences d'exclusions) n'a été entendu (par le patient) durant la première répétition. Par exemple, dans la plage de fréquences sonores, durant la première répétition, un son est entendu pour chaque fréquence de la série de fréquences sonores compris dans la plage de fréquences sonores. Selon un mode de réalisation, la plage de fréquences sonores conserve toutefois les bornes de cette plage d'exclusion.

Cette réduction de bande passante est réalisée de façon à permettre au procédé selon l'invention de converger plus rapidement et d'éviter une recherche infructueuse de seuils sur des parties du spectre pour lesquelles aucune information additionnelle ne pourrait être obtenue en raison d'une zone morte cochléaire ou de la limitation imposée par la puissance maximale délivrable par l'audiomètre qui ne permettra pas d'estimer le seuil.

Le procédé selon l'invention peut être réalisé (autrement dit : mis en œuvre) par un dispositif électronique de test audiométrique. Le dispositif électronique peut comprendre une unité centrale électronique (par exemple comprise dans un téléphone mobile ou une tablette tactile électronique) et un casque audio ou des inserts, ou des haut-parleurs pour appliquer les sons à l'oreille et le masquage de l'oreille controlatérale en transmission aérienne. En transmission osseuse, on utilise un ou plusieurs vibrateurs (non représentés). L'acquisition de l'information selon laquelle un son est entendu ou pas peut-être réalisée par un bouton sur lequel le patient appuie lorsqu'il entend un son, ou par commande vocale ou par détection d'image.

L'invention concerne aussi un dispositif électronique de test audiométrique configuré pour mettre en œuvre les étapes du procédé selon l'invention.

L'invention concerne en outre un programme d'ordinateur comprenant des instructions, exécutables par un microprocesseur ou un microcontrôleur, pour la mise en œuvre du procédé selon l'invention.

Les caractéristiques et avantages du dispositif électronique et du programme d'ordinateur sont identiques à ceux du procédé, c'est pourquoi, ils ne sont pas repris ici.

On entend qu'un élément tel que le dispositif électronique de test audiométrique, l'unité centrale, ou un autre élément est « configuré pour » réaliser une étape ou une opération, par le fait que l'élément comporte des moyens pour (autrement dit « est conformé pour » ou « est adapté pour ») réaliser l'étape ou l'opération. Il s'agit préférentiellement de moyens électroniques, par exemple un programme d'ordinateur, des données en mémoire et/ou des circuits électroniques spécialisés.

Lorsqu'une étape ou une opération est réalisée par un tel élément, cela implique généralement que l'élément comporte des moyens pour (autrement dit « est conformé pour » ou « est adapté pour ») réaliser l'étape ou l'opération. Il s'agit également par exemple de moyens électroniques, par exemple un programme d'ordinateur, des données en mémoire et/ou des circuits électroniques spécialisés.

D'autres caractéristiques et avantages de la présente invention apparaitront plus clairement à la lecture de la description détaillée qui suit comprenant des modes de réalisation de l'invention donnés à titre d'exemples nullement limitatifs et illustrés par les dessins annexés, dans lesquels :

[Fig.1] représente un dispositif électronique selon un mode de réalisation de l'invention.

[Fig.2] représente le procédé selon l'invention, dans un exemple de réalisation, mis en œuvre par le dispositif électronique de la [Fig.1].

## Description détaillée d'un exemple de réalisation de l'invention

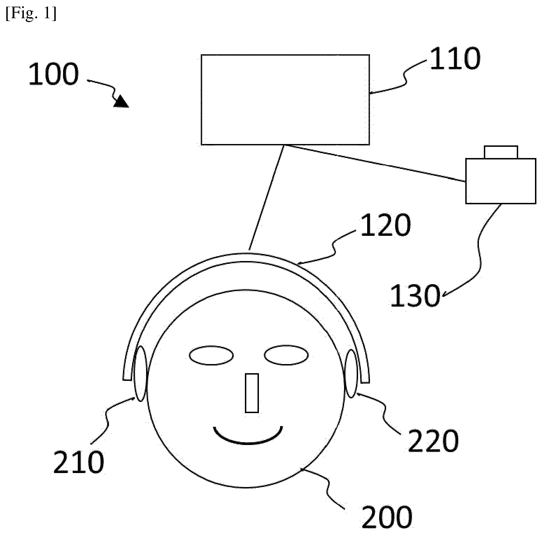

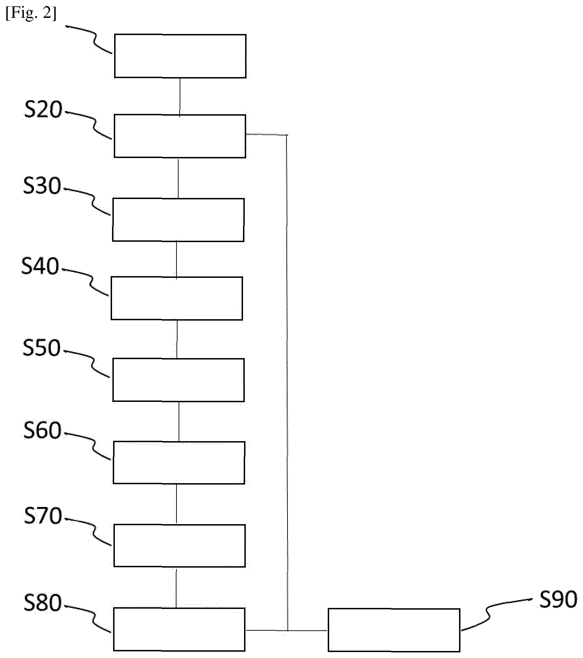

En référence aux figures 1 et 2, A l'étape S10, le procédé débute par la détermination d'une séquence, la détermination de la séquence comprenant la première répétition des étapes suivantes jusqu'à ce qu'une cinquième fréquence ait pris toutes les valeurs (autrement dit : chaque valeur) de fréquence d'une série de fréquences :

- Détermination d'un cinquième son présentant la cinquième fréquence, par exemple, 1000 Hz, et une cinquième intensité, par exemple 60 dB HL,
- Une répétition des étapes suivantes jusqu'à ce que le cinquième son ait été entendu une fois et pas entendu une fois ou jusqu'à ce que la cinquième intensité atteigne une intensité maximale, par exemple 90 dB HL ou une intensité minimale, par exemple -20 dB HL :

  - Application à l'oreille 210 du cinquième son,
  - Acquisition d'une cinquième l'information selon laquelle le cinquième son est entendu ou pas (par le patient),
  - Mise à jour de la séquence à partir du cinquième son et de la cinquième information
  - Si le cinquième son est entendu, décrémentation de la cinquième intensité d'une valeur de décrémentation, par exemple de 20 dB HL.
  - Si le cinquième son n'est pas entendu, incrémentation de la cinquième intensité d'une valeur d'incrémentation, par exemple de 10 dB HL.

- Affectation de la cinquième intensité par :

  - Si un cinquième son a été entendu (par le patient) (durant la deuxième répétition des étapes), une intensité minimum où le cinquième son a été entendu (à la cinquième fréquence) auquel on ajoute une marge d'intensité, par exemple de 10 dB HL. Si dans l'exemple ci-dessus le cinquième son est entendu à 1000 Hz et 60 dB HL et pas entendu à 50 dB HL, alors la cinquième intensité prend, à cette étape, la valeur de 70 dB HL.
  - Si le cinquième son n'a pas été entendu (par le patient) (durant la deuxième répétition des étapes), une intensité maximale, par exemple de 90 dB HL.

- Augmentation ou diminution de la cinquième fréquence d'un incrément de fréquence, (de préférence de manière à ce que la cinquième fréquence ait pris toutes les valeurs de fréquence d'une série de fréquences) par exemple 500 Hz. Dans l'exemple ci-dessus, la cinquième fréquence passe donc à 1500 Hz. Le son suivant appliqué à l'oreille est donc à 1500 Hz et 70 dB HL.

En variante, le procédé de test audiométrique est réalisé en conduction osseuse et comprend, préalablement à l'étape de détermination d'une séquence, une détermination d'un audiogramme en conduction aérienne comprenant un seuil de 40dB à 1000 Hz et 45 dB à 1500 Hz. On fait entendre au patient d'abord un son à 1000 Hz et 45 dB. Si le son n'est pas entendu, on considère que la conduction osseuse est identique à la conduction aérienne à 1000 Hz. On fait entendre ensuite au patient un son à 1500 Hz et 50 dB. Si le son est entendu, le test se poursuit comme décrit ci-dessus à l'étape S10 en prenant comme cinquième son le son d'intensité 50 dB et de fréquence 1500 Hz.

Par exemple, lorsque la cinquième fréquence est une fréquence d'extrémité (par exemple, les fréquences de 8000 Hz et 125 Hz sont des fréquences d'extrémité en conduction aérienne lorsque la série de fréquence est constituée des fréquences suivantes : 1000, 1500, 2000, 3000, 4000, 6000, 8000, 750, 500, 250 et 125 Hz) de la série de fréquences, la valeur d'incrémentation (et/ou la valeur de décrémentation, respectivement) prend une valeur inférieure, par exemple 10 dB HL, à la valeur d'incrémentation (et/ou la valeur de décrémentation, respectivement) lorsque la fréquence est une autre fréquence (qu'une fréquence d'extrémité).

Lorsque la cinquième intensité dépasse un cinquième seuil, par exemple 80 dB HL, la valeur d'incrémentation prend une valeur inférieure, par exemple 5 dB HL, à la valeur d'incrémentation lorsque la cinquième intensité est en dessous du cinquième seuil.

La série de fréquence est par exemple constituée des fréquences suivantes : 1000, 1500, 2000, 3000, 4000, 6000, 8000, 750, 500, 250 et 125 Hz.

Par exemple, à l'étape S10, durant la première répétition, aucun son par exemple n'est entendu par le patient 200 à 6000 Hz et à 8000 Hz. Un son est par contre entendu aux fréquences 1000, 1500, 2000, 3000, 4000, 750, 500, 250 et 125 Hz.

A l'étape S20, il est vérifié si une condition est remplie. La condition est remplie si :

- L'amplitude d'un intervalle estimé par le processus gaussien est inférieure à une valeur seuil (par exemple 6 dB HL),
- Le nombre de fois dépasse un seuil de répétition (par exemple compris entre 15 et 100 fois).

Si la condition est remplie, alors le procédé se termine à l'étape S90.

Si la condition n'est pas remplie, alors les étapes suivantes sont réalisées :

- A l'étape S30, détermination d'un deuxième son ayant une deuxième fréquence, par exemple 4500 Hz, et une deuxième intensité, par exemple 105 dB HL, à partir d'un processus gaussien prenant la séquence comme données observées dans une plage de fréquences sonores s'entendant entre 125Hz et 6000Hz (puisque à l'étape S10, durant la première répétition, aucun son par exemple n'est entendu par le patient 200 à 6000 Hz et à 8000 Hz).
- A l'étape S40, détermination s'il existe, d'un troisième son de la séquence présentant une troisième intensité, par exemple 90 dB HL, inférieure à la deuxième intensité et supérieure à un deuxième seuil par exemple égale à 80 dB HL et une troisième fréquence, par exemple 4000 Hz, pour laquelle la différence entre la troisième fréquence et la deuxième fréquence est inférieure à un seuil de fréquence par exemple égale à 500 Hz ( en Hertz, notés Hz), et pour lequel la troisième intensité est maximum parmi tous les sons de la séquence dont la fréquence a une différence avec la deuxième fréquence inférieure au seuil de fréquence (par exemple la séquence comporte, entre 4000 Hz et 6000 Hz, un son à 4000 Hz et 90 dB HL un son à 6000Hz non entendu. Le troisième son est le son à 4000 Hz et 90 dB HL),
- A l'étape S50, application à l'oreille 210 d'un quatrième son ayant une quatrième fréquence et une quatrième intensité, où :

  - Si la deuxième intensité est supérieure au deuxième seuil, par exemple égale à 80 dB HL, et s'il existe un troisième son, la quatrième fréquence est égale à la deuxième fréquence, (dans notre exemple, 4500 Hz), et la quatrième intensité est égale à la troisième intensité (dans notre exemple, 90 dB HL) augmentée d'un incrément prédéfini, par exemple 5 dB HL. Donc la quatrième intensité est égale à 95 dB HL.
  - Sinon (c'est-à-dire, la deuxième intensité est inférieure au deuxième seuil ou il n'existe pas de de troisième son) alors le quatrième son est identique au deuxième son.

- A l'étape S60, acquisition d'une quatrième information selon laquelle le quatrième son est entendu ou pas (par le patient)
- A l'étape S70, mise à jour de la séquence à partir de la quatrième information et du quatrième son.
- A l'étape S80, le procédé se poursuit par l'étape S20.

Par exemple, le processus gaussien est mis en œuvre comme décrit dans l'article de Schlittenlacher J, Turner RE, Moore BCJ. « Audiogram estimation using Bayesian active learning » (J Acoust Soc Am. 2018;144(1):421). Notamment le processus a les caractéristiques suivantes.

Le procédé selon l'invention peut être réalisé (autrement dit : mise en œuvre) par un dispositif électronique 100 de test audiométrique. Le dispositif électronique 100 peut comprendre une unité centrale 110 et un casque audio 120 pour appliquer les sons à l'oreille 210 et le masquage de l'oreille controlatérale 220 en transmission aérienne d'un patient 200. En transmission osseuse, on utilise un ou plusieurs vibrateurs (non représenté) en combinaison avec un casque audio en transmission aérienne pour le masquage de l'oreille controlatérale. L'acquisition de l'information selon laquelle un son est entendu ou pas peut-être réalisée par un bouton 130 sur lequel le patient appuie lorsqu'il entend un son.

**Revendications**

1. Procédé de test audiométrique d'une oreille (210) **caractérisé en ce qu'**il comporte les étapes suivantes :

- Détermination (S10) d'une séquence comprenant:

  - Des premiers sons appliqués à l'oreille (210),
  - Pour chacun des premiers sons, une première information selon laquelle les premiers sons sont entendus ou pas,

- Répétition des étapes suivantes un nombre de fois jusqu'à ce qu'une condition soit remplie (S20) :

  - Détermination (S30) d'un deuxième son ayant une deuxième fréquence, et une deuxième intensité, à partir d'un processus gaussien prenant la séquence comme données observées,
  - Détermination (S40), s'il existe, d'un troisième son de la séquence présentant une troisième intensité, inférieure à la deuxième intensité et supérieure à un deuxième seuil et présentant une troisième fréquence, la différence entre la troisième fréquence et la deuxième fréquence étant inférieure à un seuil de fréquence, et la troisième intensité étant maximum parmi tous les sons de la séquence dont la fréquence à une différence avec la deuxième fréquence inférieure au seuil de fréquence,
  - Application (S50) à l'oreille (210) d'un quatrième son ayant une quatrième fréquence et une quatrième intensité, où :

    - Si la deuxième intensité est supérieure au deuxième seuil, et s'il existe un troisième son, la quatrième fréquence est égale à la deuxième fréquence, et la quatrième intensité est égale à la troisième intensité augmentée d'un incrément prédéfini,
    - Sinon, alors le quatrième son est identique au deuxième son.

  - Acquisition (S60) d'une quatrième information selon laquelle le quatrième son est entendu ou pas,
  - Mise à jour (S70) de la séquence à partir de la quatrième information et du quatrième son.

2. Procédé de test audiométrique selon la revendication précédente dans lequel la condition est remplie si :

  - Une amplitude d'un intervalle estimé par le processus gaussien est inférieure à une valeur seuil, ou
  - Le nombre de fois dépasse un seuil de répétition.

3. Procédé de test audiométrique selon l'une quelconque des revendications 1 ou 2 dans lequel l'étape de détermination d'une séquence comprend la répétition des étapes suivantes jusqu'à ce qu'une cinquième fréquence ait pris toutes les valeurs de fréquence d'une série de fréquences :

  - Détermination d'un cinquième son présentant la cinquième fréquence, et une cinquième intensité,
  - Application à l'oreille (210) du cinquième son,
  - Acquisition d'une cinquième information selon laquelle le cinquième son est entendu ou pas (par le patient),
  - Mise à jour de la séquence à partir du cinquième son et de la cinquième information.

4. Procédé de test audiométrique selon l'une quelconque des revendications 1 ou 2 dans lequel l'étape de détermination d'une séquence comprend la première répétition des étapes suivantes jusqu'à ce qu'une cinquième fréquence ait pris toutes les valeurs de fréquence d'une série de fréquences :

  - Détermination d'un cinquième son présentant la cinquième fréquence, et une cinquième intensité,
  - Une répétition des étapes suivantes jusqu'à ce que le cinquième son ait été entendu une fois et pas entendu une fois ou jusqu'à ce que la cinquième intensité atteigne une intensité maximale, ou une intensité minimale :

    - Application à l'oreille (210) du cinquième son,
    - Acquisition d'une cinquième l'information selon laquelle le cinquième son est entendu ou pas,
    - Mise à jour de la séquence à partir du cinquième son et de la cinquième information,
    - Si le cinquième son est entendu, décrémentation de la cinquième intensité d'une valeur de décrémentation.
    - Si le cinquième son n'est pas entendu, incrémentation de la cinquième intensité d'une valeur d'incrémentation.

  - Affectation de la cinquième intensité par :

    - Si le cinquième son a été entendu, une intensité minimum où le cinquième son a été entendu auquel on ajoute une marge d'intensité,
- Si le cinquième son n'a pas été entendu, l'intensité maximale,

- Augmentation ou diminution de la cinquième fréquence d'un incrément de fréquence.

5. Procédé de test audiométrique selon la revendication précédente, dans lequel, lorsque la cinquième fréquence est une fréquence d'extrémité de la série de fréquences, la valeur d'incrémentation prend une valeur inférieure, à la valeur d'incrémentation lorsque la fréquence est une autre fréquence.

6. Procédé de test audiométrique selon l'une quelconque des revendications 1 ou 2 , dans lequel le test audiométrique est réalisé en conduction osseuse et comprenant, préalablement à l'étape de détermination d'une séquence :

- Une détermination d'un audiogramme en conduction aérienne comprenant une intensité aérienne pour chaque fréquence d'une série de fréquence (l'intensité aérienne pour chaque fréquence est l'intensité minimum où le son est entendu à cette fréquence),
- Une initialisation d'un état à une première valeur, (l'état est par exemple mémorisé en mémoire)

et dans lequel l'étape de détermination d'une séquence comprend la première répétition des étapes suivantes jusqu'à ce qu'une cinquième fréquence ait pris toutes les valeurs de fréquence de la série de fréquences, la cinquième fréquence étant initialisée à la première fréquence de la série de fréquence :

- Si l'état à la première valeur :

- Détermination d'un cinquième son présentant la cinquième fréquence, et une cinquième intensité égale à l'intensité aérienne pour la cinquième fréquence dans l'audiogramme en conduction aérienne auquel on ajoute une intensité additionnelle,
- Application à l'oreille (210) du cinquième son par conduction osseuse,
- Acquisition d'une cinquième l'information selon laquelle le cinquième son est entendu ou pas,
- Si le cinquième son est entendu, affectation de l'état par une deuxième valeur.

- Si l'état à la deuxième valeur :

- Une répétition des étapes suivantes jusqu'à ce que le cinquième son ait été entendu une fois et pas entendu une fois ou jusqu'à ce que la cinquième intensité atteigne une intensité maximale, ou une intensité minimale :

- Application à l'oreille (210) du cinquième son,
- Acquisition d'une cinquième l'information selon laquelle le cinquième son est entendu ou pas,
- Mise à jour de la séquence à partir du cinquième son et de la cinquième information,
- Si le cinquième son est entendu, décrémentation de la cinquième intensité d'une valeur de décrémentation.
- Si le cinquième son n'est pas entendu, incrémentation de la cinquième intensité d'une valeur d'incrémentation.

- Affectation de la cinquième intensité par :

- Si le cinquième son a été entendu, une intensité minimum où le cinquième son a été entendu auquel on ajoute une marge d'intensité,
- Si le cinquième son n'a pas été entendu, l'intensité maximale,

- Augmentation de la cinquième fréquence d'un incrément de fréquence.

7. Procédé de test audiométrique selon l'une quelconque des revendications 4 à 6, dans lequel, lorsque la cinquième intensité dépasse un cinquième seuil, la valeur d'incrémentation prend une valeur inférieure, à la valeur d'incrémentation lorsque la cinquième intensité est en dessous du cinquième seuil.

8. Procédé de test audiométrique selon l'une quelconque des revendications 4 à 7 dans lequel, durant l'étape de détermination du deuxième son, le deuxième son est déterminé, à partir du processus gaussien, dans une plage de fréquences sonores, et la plage de fréquences sonores exclue une plage de fréquence d'exclusions où aucun son n'a été entendu durant la première répétition.

9. Procédé de test audiométrique selon l'une quelconque des revendications 3 à 8 dans lequel la série de fréquence est constituée des fréquences suivantes : 1000, 1500, 2000, 3000, 4000, 6000, 8000, 750, 500, 250 et 125 Hz en conduction aérienne et 1000, 1500, 2000, 3000, 4000, 6000, 750, 500 et 250 Hz en conduction osseuse.

10. Dispositif électronique (100) de test audiométrique configuré pour mettre en œuvre les étapes du procédé selon l'une quelconque des revendications 1 à 9.

11. Programme d'ordinateur comprenant des instructions, qui conduisent le dispositif selon la revendication 10 à exécuter les étapes du procédé selon l'une quelconque des revendications 1 à 9.

**Patentansprüche**

1. Audiometrisches Testverfahren eines Ohrs (210) **dadurch gekennzeichnet, dass** es die folgenden Schritte umfasst:

   - Bestimmung (S10) einer Sequenz aufweisend:

     ◦ die am Ohr (210) angebrachten ersten Klänge,
     ◦ für jeden der ersten Klänge eine erste Information, dass die ersten Klänge wohl oder nicht wahrgenommen werden;

   - mehrfache Wiederholung der folgenden Schritte, bis eine Bedingung erfüllt ist (S20):

     ◦ Bestimmung (S30) eines zweiten Klangs mit einer zweiten Frequenz und einer zweiten Intensität aus einem Gauß-Prozess mit Sequenz als beobachtete Daten;
     ◦ Bestimmung (S40), wenn vorliegend, eines dritten Klangs der Sequenz mit einer dritten Intensität, kleiner als die zweite Intensität und größer als ein zweiter Schwellenwert und mit einer dritten Frequenz, wobei die Differenz zwischen der dritten und der zweiten Frequenz kleiner als ein Frequenzschwellenwert ist; und wobei die dritte Intensität unter allen Klängen der Sequenz, deren Frequenz einen Unterschied von der zweiten Frequenz kleiner als der Frequenzschwellenwert hat, maximal ist;
     ◦ Anwendung (S50) am Ohr (210) von einem vierten Klang mit einer vierten Frequenz und einer vierten Intensität, wobei:

       ▪ wenn die zweite Intensität größer als der zweite Schwellenwert ist, und wenn ein dritter Klang vorliegend ist, die vierte Frequenz gleich der zweiten Frequenz ist und die vierte Intensität gleich der dritten Intensität, zuzüglich eines vordefinierten Inkrements, ist;
       ▪ ansonsten ist der vierte Klang identisch mit dem zweiten Klang.

   - Erfassung (S60) einer vierten Information, dass der vierte Klang wohl oder nicht wahrgenommen wird;
   - Aktualisierung (S70) der Sequenz aus der vierten Information und dem vierten Klang.

2. Audiometrisches Testverfahren nach dem vorhergehenden Anspruch, wobei die Bedingung erfüllt ist, wenn:

   - eine durch den Gauß-Prozess bestimmte Amplitude eines Intervalls kleiner als ein Schwellenwert ist oder
   - die Anzahl der Male größer als ein Wiederholungsschwellenwert ist.

3. Audiometrisches Testverfahren nach einem der Ansprüche 1 oder 2, wobei der Schritt zur Bestimmung einer Sequenz die Wiederholung, bis eine fünfte Frequenz alle Frequenzwerte einer Frequenzreihe angenommen hat, der folgenden Schritte umfasst:

   - Bestimmung eines fünften Klangs mit der fünften Frequenz und einer fünften Intensität,
   - Anwendung am Ohr (210) des fünften Klangs,
   - Erfassung einer fünften Information, dass der fünfte Klang wohl oder nicht (vom Patienten) wahrgenommen wird,
   - Aktualisierung der Sequenz aus dem fünften Klang und der fünften Information.

4. Audiometrisches Testverfahren nach einem der Ansprüche 1 oder 2, wobei der Schritt zur Bestimmung einer Sequenz die erste Wiederholung, bis eine fünfte Frequenz alle Frequenzwerte einer Frequenzreihe angenommen hat, der folgenden Schritte umfasst:

- Bestimmung eines fünften Klangs mit der fünften Frequenz und einer fünften Intensität,
- Wiederholung der folgenden Schritte, bis der fünfte Klang einmal wahrgenommen und einmal nicht wahrgenommen wurde oder bis die fünfte Intensität eine maximale Intensität oder eine minimale Intensität erreicht hat:

    ◦ Anwendung am Ohr (210) des fünften Klangs,
    ◦ Erfassung einer fünften Information, dass der fünfte Klang wohl oder nicht wahrgenommen wird,
    ◦ Aktualisierung der Sequenz aus dem fünften Klang und der fünften Information,
    ◦ wenn der fünfte Klang wahrgenommen wird, Dekrement der fünften Intensität um einen Dekrementwert,
    ◦ wenn der fünfte Klang nicht wahrgenommen wurde, Inkrement der fünften Intensität um einen Inkrementwert,

- Zuweisung der fünften Intensität durch:

    ◦ wenn der fünfte Klang wahrgenommen wurde, eine Mindestintensität, bei der der fünfte Klang wahrgenommen wurde, zu der eine Intensitätspanne hinzufügt wird;
    ◦ wenn der fünfte Klang nicht wahrgenommen wurde, die maximale Intensität,

- Erhöhung oder Abnahme der fünften Frequenz um ein Frequenzinkrement.

5. Audiometrisches Testverfahren nach dem vorhergehenden Anspruch, wobei, wenn die fünfte Frequenz eine Endfrequenz der Frequenzreihe ist, der Inkrementwert einen Wert kleiner als der Inkrementwert annimmt, wenn die Frequenz eine andere Frequenz ist.

6. Audiometrisches Testverfahren nach einem der Ansprüche 1 oder 2, wobei der audiometrische Test in Knochenleitung durchgeführt wird und, vor dem Schritt zur Bestimmung einer Sequenz, umfasst:

- Bestimmung eines Audiogramms in Luftleitung mit einer Luftintensität für jede Frequenz einer Frequenzreihe (die Luftintensität für jede Frequenz ist die Mindestintensität, wobei der Klang bei dieser Frequenz wahrgenommen wird);
- Initialisierung eines Zustands an einen ersten Wert (z. B. wird der Zustand im Speicher gespeichert)

und wobei der Schritt zur Bestimmung einer Sequenz die erste Wiederholung der folgenden Schritte umfasst, bis eine fünfte Frequenz alle Frequenzwerte der Frequenzreihe angenommen hat, wobei die fünfte Frequenz mit der ersten Frequenz der Frequenzreihe initialisiert wird:

- wenn der Zustand den ersten Wert hat:

    ◦ Bestimmung eines fünften Klangs mit der fünften Frequenz und einer fünften Intensität, die gleich der Luftintensität für die fünfte Frequenz im Audiogramm in Luftleitung ist, dem eine zusätzliche Intensität hinzugefügt wird;
    ◦ Anwendung am Ohr (210) des fünften Klangs in Knochenleitung,
    ◦ Erfassung einer fünften Information, dass der fünfte Klang wohl oder nicht wahrgenommen wird,
    ◦ wenn der fünfte Klang wahrgenommen wird, Zuordnung des Zustands durch einen zweiten Wert,

- wenn der Zustand den zweiten Wert hat:

    ◦ Wiederholung der folgenden Schritte, bis der fünfte Klang einmal wahrgenommen und einmal nicht wahrgenommen wurde oder bis die fünfte Intensität eine maximale oder minimale Intensität erreicht hat:

        ▪ Anwendung am Ohr (210) des fünften Klangs,
        ▪ Erfassung einer fünften Information, dass der fünfte Klang wohl oder nicht wahrgenommen wird,
        ▪ Aktualisierung der Sequenz aus dem fünften Klang und der fünften Information,
        ▪ wenn der fünfte Klang wahrgenommen wird, Dekrement der fünften Intensität um einen Dekrementwert,

11

■ wenn der fünfte Klang nicht wahrgenommen wurde, Inkrement der fünften Intensität um einen Inkrementwert,

◦ Zuweisung der fünften Intensität durch:

■ wenn der fünfte Klang wahrgenommen wurde, eine Mindestintensität, bei der der fünfte Klang wahrgenommen wurde, zu der eine Intensitätspanne hinzufügt wird;
■ wenn der fünfte Klang nicht wahrgenommen wurde, die maximale Intensität,

- Erhöhung der fünften Frequenz um einen Frequenzschritt.

7. Audiometrisches Testverfahren nach einem der Ansprüche 4 bis 6, wobei, wenn die fünfte Intensität größer als ein fünfter Schwellenwert ist, der Inkrementwert einen Wert kleiner als der Inkrementwert annimmt, wenn die fünfte Intensität kleiner als der fünfte Schwellenwert ist.

8. Audiometrisches Testverfahren nach einem der Ansprüche 4 bis 7, wobei im Schritt zur Bestimmung des zweiten Klangs der zweite Klang aus dem Gauß-Prozess in einem Schallfrequenzbereich bestimmt wird, und der Schallfrequenzbereich einen Frequenzbereich von Ausschlüssen ausschließt, bei dem während der ersten Wiederholung kein Klang wahrgenommen wurde.

9. Audiometrisches Testverfahren nach einem der Ansprüche 3 bis 8, wobei die Frequenzreihe aus folgenden Frequenzen besteht: 1000, 1500, 2000, 3000, 4000, 6000, 8000, 750, 500, 250 und 125 Hz in Luftleitung und 1000, 1500, 2000, 3000, 4000, 6000, 750, 500 und 250 Hz in Knochenleitung.

10. Elektronische, audiometrische Testvorrichtung (100) zur Durchführung der Verfahrensschritte nach einem der Ansprüche 1 bis 9.

11. Computerprogramm umfassend Anweisungen, die die Vorrichtung nach Anspruch 10 dazu veranlassen, die Verfahrensschritte nach einem der Ansprüche 1 bis 9 durchzuführen.


**Claims**

1. Method for audiometry testing of an ear (210), **characterized in that** it comprises the following steps:

- Determination (S10) of a sequence comprising:

- From the first sounds applied to the ear (210),
- For each of the first sounds, initial information as to whether the first sounds are heard or not,

- Repeat the following steps a number of times until a condition is met (S20):

- Determination (S30) of a second sound having a second frequency, and a second intensity, from a Gaussian process taking the sequence as observed data,
- Determination (S40), if any, of a third sound in the sequence having a third intensity, lower than the second intensity and higher than a second threshold, and having a third frequency, the difference between the third frequency and the second frequency being lower than a frequency threshold, and the third intensity being maximum among all the sounds in the sequence whose frequency has a difference with the second frequency lower than the frequency threshold,
- Application (S50) to the ear (210) of a fourth sound having a fourth frequency and a fourth intensity, whereby:

- If the second intensity is greater than the second threshold, and if a third sound exists, the fourth frequency is equal to the second frequency, and the fourth intensity is equal to the third intensity increased by a predefined increment,
- If not, then the fourth sound is identical to the second sound.

- Acquisition (S60) of fourth information on whether or not the fourth sound is heard,
- Update (S70) of the sequence based on the fourth item of information and the fourth sound.

2. Audiometry test method according to the preceding claim in which the condition is fulfilled if:

   - An interval amplitude estimated by the Gaussian process is below a threshold value, or
   - The number of times exceeds a repetition threshold.

3. An audiometry test method according to any one of claims 1 or 2 in which the step of determining a sequence comprises repeating the following steps until a fifth frequency has taken on all the frequency values of a series of frequencies:

   - Determination of a fifth sound with a fifth frequency and a fifth intensity,
   - Application of the fifth sound to the ear (210),
   - Acquisition of a fifth piece of information, depending on whether or not the fifth sound is heard (by the patient),
   - The sequence is updated with the fifth sound and the fifth piece of information.

4. Audiometry test method according to any one of claims 1 or 2 in which the step of determining a sequence comprises the first repetition of the following steps until a fifth frequency has taken on all the frequency values of a series of frequencies:

   - Determination of a fifth sound with a fifth frequency and a fifth intensity,
   - Repeat the following steps until the fifth sound has been heard once and not heard once, or until the fifth intensity reaches maximum intensity, or minimum intensity:

     - Application of the fifth sound to the ear (210),
     - Acquisition of a fifth piece of information as to whether the fifth sound is heard or not,
     - The sequence is updated with the fifth sound and the fifth piece of information,
     - If the fifth sound is heard, decrement the fifth intensity by one decrement value.
     - If the fifth sound is not heard, increment the fifth intensity by one incremental value.

   - Allocation of the fifth intensity by:

     - If the fifth sound has been heard, a minimum intensity where the fifth sound has been heard to which an intensity margin is added,
     - If the fifth sound has not been heard, set the intensity to maximum,

   - Increase or decrease the fifth frequency by one frequency increment.

5. Audiometry test method according to the preceding claim, in which, when the fifth frequency is an end frequency of the frequency series, the increment value takes on a lower value than the increment value when the frequency is another frequency.

6. Audiometry test method according to any one of claims 1 or 2, in which the audiometry test is performed in bone-conduction and comprising, prior to the step of determining a sequence:

   - A determination of an air-conduction audiogram comprising an air intensity for each frequency in a frequency series (the air intensity for each frequency is the minimum intensity at which sound is heard at that frequency),
   - Initialization of a state to a first value (for example, the state is stored in memory)

   and wherein the step of determining a sequence comprises the first repetition of the following steps until a fifth frequency has taken all frequency values of the frequency series, the fifth frequency being initialized to the first frequency of the frequency series:

   - If the state has the first value:

     - Determination of a fifth sound with the fifth frequency, and a fifth intensity equal to the air intensity for the fifth frequency in the air-conduction audiogram, to which an additional intensity is added,
     - Application of the fifth bone-conduction sound to the ear (210),
     - Acquisition of a fifth piece of information as to whether the fifth sound is heard or not,
     - If the fifth sound is heard, the state is assigned a second value.

- If the state has the second value:

    - Repeat the following steps until the fifth sound has been heard once and not heard once, or until the fifth intensity reaches maximum intensity, or minimum intensity:

        - Application of the fifth sound to the ear (210),
        - Acquisition of a fifth piece of information as to whether the fifth sound is heard or not,
        - The sequence is updated with the fifth sound and the fifth piece of information,
        - If the fifth sound is heard, decrement the fifth intensity by one decrement value.
        - If the fifth sound is not heard, increment the fifth intensity by one incremental value.

    - Allocation of the fifth intensity as follows:

        - If the fifth sound has been heard, a minimum intensity where the fifth sound has been heard to which an intensity margin is added,
        - If the fifth sound has not been heard, set the intensity to maximum,

    - Increase the fifth frequency by one frequency increment.

7. Audiometry test method according to any one of claims 4 to 6, in which, when the fifth intensity exceeds a fifth threshold, the increment value takes on a lower value than the increment value when the fifth intensity is below the fifth threshold.

8. Audiometry test method according to any one of claims 4 to 7 in which, during the step of determining the second sound, the second sound is determined, from the Gaussian process, in a sound frequency range, and the sound frequency range excludes an exclusion frequency range where no sound was heard during the first repetition.

9. Audiometry test method according to any one of claims 3 to 8 in which the frequency series consists of the following frequencies: 1000, 1500, 2000, 3000, 4000, 6000, 8000, 750, 500, 250 and 125 Hz in air-conduction and 1000, 1500, 2000, 3000, 4000, 6000, 750, 500 and 250 Hz in bone-conduction.

10. Electronic audiometric testing device (100) configured to implement the steps of the process according to any one of claims 1 to 9.

11. Computer program comprising instructions that cause the device according to claim 10 to perform the steps of the process according to any of claims 1 to 9.

[Fig. 1]

[Fig. 2]

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2010257128 A1

**Littérature non-brevet citée dans la description**

- **TREVOR J LARSEN et al.** Accelerating Psychometric Screening Tests With Bayesian Active Differential Selection. CORNELL UNIVERSITY LIBRARY, February 2020
- **COX MARCO et al.** Bayesian Pure-Tone Audiometry Through Active Learning Under Informed Priors. *Frontiers in digital health*, August 2021

- **SONG XD** ; **WALLACE BM** ; **GARDNER JR** ; **LEDBETTER NM** ; **WEINBERGER KQ** ; **BARBOUR DL. FAST**. Continuous Audiogram Estimation using Machine Learning. *Ear Hear.*, 2015, vol. 36 (6), e326-35
- **SCHLITTENLACHER J** ; **TURNER RE** ; **MOORE BCJ.** Audiogram estimation using Bayesian active learning. *J Acoust Soc Am.*, 2018, vol. 144 (1), 421